# EUROPEAN PATENT APPLICATION

(11) **EP 1 543 807 A1**
(43) Date of publication of application: **22.06.2005**
(21) Application number: 03029227.0
(22) Date of filing: 18.12.2003
(51) Int. Cl.: A61F 13/58, A61F 13/60

(54) **Closure tape tab for an absorbent article, prelaminated closure tape and method of manufacturing the closure tape tab**

(71) Applicant: 3M Innovative Properties Company, St. Paul, MN 55133-3427 (US)
(72) Inventor: Selen, Peter Gertud, 5993 PG Maasbree (NL); Guenther, Werner, 41462 Neuss (DE); Jung, Dieter, 47447 Moers (DE)
(74) Representative: VOSSIUS & PARTNER

(57) **Abstract**

The present invention relates to a closure tape tab for an absorbent article, particularly for a disposable diaper, for fastening the article on the body of a person. The closure tape tab comprises a proximal end portion and a distal end portion being connected by an inner tab portion, wherein the inner tab portion has a first major surface and a second major surface. The proximal and distal end portions are connected to the inner tab portion at the first major surface thereof such that opposing ends of the proximal and distal end portions are spaced apart from each other. An anti-adhesive means is provided at at least a part of the first major surface of the inner tab portion in the space. The present invention furthermore relates to a prelaminated closure tape, preferably in a stable roll, from which such closure tape tabs can be cut. The closure tape of the present invention is adapted to be level-wound on a roll so that an increased amount of tape can be stored on the roll.

## Description

### Field of the Invention

The present invention relates to a closure tape tab for an absorbent article, particularly for a disposable diaper or an adult incontinent article, for fastening of the article on the body of a person. The invention furthermore relates to a prelaminated closure tape, preferably in a stable roll, from which closure tape tabs can be cut. Moreover, the present invention relates to a method of manufacturing such a closure tape tab.

### Background of the Invention

A disposable diaper typically has a thin, flexible, stretchy, low density polyethylene back sheet film, an absorbable core on the inside of the back sheet film and a porous top sheet overlying the core. The diaper is positioned at the crotch of the wearer, two ends of the diaper extending, respectively, towards the front and back. Adjacent edges of the diaper at each side are then either positioned adjacent to each other or overlapped. A closure tape tab is used to close the diaper.

Such a closure tape tab is typically anchored to the diaper to one of its end portions (proximal end portion) by means of, for example, a pressure sensitive adhesive layer. The opposite end portion (distal end portion) of the closure tape tab comprises a fastening means (e.g., a pressure sensitive adhesive layer or a mechanical fastener component) to close the diaper around the wearer's body and fasten it on the body.

EP-A-0 247 855 relates to a tape fastener laminate on a roll comprising a pressure sensitive adhesive fastener, a central elastic part, a two-part release coated fastening tape and a release tape underneath having an adhesive layer which is covered by a non-tacky film.

EP-A-0 257 752 describes a fastening tape for diaper's wound convolutedly to a roll for storage. The fastening tape comprises a one-piece backing, a release tape and a pressure sensitive adhesive fastener. The backing has a release coat to allow for unwinding the tape from the roll.

EP-A-0 456 472 discloses an adhesive closure tape having a surface release directly adjacent to an adhesive, wherein an adhesive strip may be coated on a previously release coated area so that the laminate can be wound into a roll.

EP-A-0 482 383 relates to a composite laminate adhesive tape coiled endlessly in roll form, wherein in cross-section the composite laminated adhesive tape is folded in z-form, with the top, slanted and bottom bars of the z-shape being formed by separate first, second and third tape sections of first fastening tape, second central tape and third fastening tape, respectively.

WO 96 02218 describes a two-part fastening tape consisting of a fastening part and a release part and having a pressure sensitive adhesive fastener to be received by the release part when folded over.

EP-A-0 891 760 relates to a closure tape for an absorbent article comprising a backing bearing an adhesive layer, a fastening means and a stretchable elastic sheet. The backing is essentially non-elastic and/or essentially non-extensible. The support sheet comprising the backing and the adhesive layer exhibits one or more incisions in the area of the elastic sheet with at least some of the incisions extending in machine direction over the full width of the backing and the end portion being separated from the incision closest to the end portion by a sufficiently large distance to prevent the incisions essentially from opening and attaching the end portion to the outside surface of the diaper when bending the remaining part of the closure tape to contact the inside of the diaper.

EP-A-1 256 332 discloses a similar construction as the afore-mentioned EP-A-0 891 760, wherein the ratio of the extension of the elastic sheet in the cross-direction over the length of the closure tape prior to fastening it to the absorbent article is between 0.1 and 0.9.

EP-A-0 941 730 describes a fastening tape for diapers with an elastic component as well as with a mechanical fastener, wherein the fastening tape has a backing treated with a release lining. A covering film is arranged to partially cover the adhesive on the lower side of the fastening tape.

EP-A-1 002 846 describes a fastening tape comprising a backing having a fibrous layer of woven fibers or of non-woven fibers of a thermoplastic polymer, wherein the backing comprises on the fibrous layer a silicon release layer and the side of the backing opposite to the side comprising the silicon release layer comprises a pressure-sensitive adhesive layer.

WO 01/68025 describes an elastic laminate tape fastener having a pressure sensitive adhesive element and an expandable fibrous layer which is bonded to an elastic film pointwise to provide for a corrugated structure of the fibrous layer. When stretching the elastic film, the fibrous layer will be brought to its original flat shape and length without stretching it.

While these known fastening tape tabs are widely used and provide several advantages, there remains a need in the field for further improvement, particularly in handling a prelaminated closure tape to form a stable roll and in manufacturing the closure tape tabs. It is an object of the present invention to meet this need.

### Summary of the Invention

In accordance with one aspect of the present invention there is provided a closure tape tab for an absorbent article, particularly for a disposable diaper, for fastening of the article on the body of a person. The closure tape tab comprises a proximal end portion and a distal end portion which are connected by an inner tab portion. The inner tab portion has a first major surface and an opposite second major surface, wherein the proximal and distal end portions are connected to the inner tab portion at the first major surface thereof, such that opposing ends of the proximal and distal end portions are spaced apart from each other. An anti-adhesive means is provided at at least a part of the first major surface of the inner tab portion in the space.

Preferably, the anti-adhesive means extends in the space at least from adjacent the end of the proximal end portion towards the opposing end of the distal end portion. More preferably, the anti-adhesive means essentially covers the space inbetween the opposing ends of the proximal and distal end portions. It may also be preferable that the anti-adhesive means (paper, film, strip, liner or coating) extends at least partially over one or both of the opposing ends of the proximal and distal end portions. In the latter embodiment it would be advantageous to provide the anti-adhesive means at least as far over the proximal and/or distal end portion as the inner tab portion overlaps the respective end portions or at least over the sites where the inner tab portion is connected to the proximal and/or distal end portions.

According to any of the embodiments of the present invention, the closure tape tab may further comprise a release tape attached to the proximal end portion or the second major surface of the inner tab portion for securing of the closure tape tab to the absorbent article in a so-called Y-type fashion. The release tape preferably extends at least partially along the second major surface of the inner tab portion. In case a release tape is used, the anti-adhesive means extends in the space along the first major surface of the inner tab portion at least as far as the release tape extends on the second major surface of the inner tab portion.

It is also possible to provide the proximal and distal end portions as well as the anti-adhesive means from a single sheet of material, e.g., as an integral element, wherein the anti-adhesive means is formed as an intermediate part connecting the proximal and distal end portions. More precisely, the closure tape tab according to this embodiment comprises a continuous tape member having at least a proximal end portion and a distal end portion. The proximal and distal end portions are connected to an inner tab portion, which inner tab portion has a first major surface and an opposite second major surface. The proximal and distal end portions are connected to the inner tab portion at the first major surface thereof The continuous tape member provides an anti-adhesive means for said inner tab portion and comprises at least one line of weakness in the area of the inner tab portion.

By means of the line of weakness the intermediate part can be broken or opened so that the closure tape tab is usable. Alternatively, the intermediate part may be removable from the closure tape tab so as to expose the first major surface of the inner tab portion. To this end it may be advantageous to provide two lines of weakness. A first line of weakness (e.g., perforation) may be provided between the proximal end portion and an intermediate part, and a second line of weakness may be provided between the distal end portion and the intermediate part. Thus, the anti-adhesive means may be formed from the same element as the proximal and distal end portions, wherein such an integral construction can be adapted to allow for removing the anti-adhesive means. Furthermore, the intermediate part should be free of adhesive which could be achieved, e.g. by stripcoating adhesive only along the proximal and distal end portions with a gap along the intermediate part inbetween. Instead of a line of weakness (perforation) a sheet of material forming the proximal and distal end portions and the intermediate part can also be cut across its whole width provided that it still adheres sufficiently to the first major surface of the inner tab portion, while being easily removable from (e.g., low friction slidable) the inner tab portion.

In accordance with another aspect of the present invention the closure tape tab comprises a proximal end portion and a distal end portion which are connected by an inner tab portion. The inner tab portion has a first major surface and an opposite second major surface, wherein the proximal end portion is connected to the inner tab portion at the second major surface thereof, such that the proximal end portion extends over at least a substantial portion of the inner tab portion. The distal end portion is connected to the inner tab portion at the first major surface thereof such that at least a portion thereof remains exposed. An anti-adhesive means is provided at at least a part of the exposed portion of the first major surface of the inner tab portion. Preferably, the anti-adhesive means extends over substantially the entire length of the exposed portion of the inner tab portion. The expression "over at least a substantial portion" appearing above and below means that the proximal end portion extends at least so far over the second major surface of the inner tab portion that the proximal end portion and the inner tab portion can be secured to each other reliably.

Furthermore, in accordance with the present invention (independently of the various aspects thereof) the anti-adhesive means can be an anti-adhesive paper, anti-adhesive film, a cover strip or a liner. Alternatively, the anti-adhesive means may be an anti-adhesive coating, preferably a silicon coating. It is also possible that the inner tab portion comprises an elastic allowing for some extensibility of the closure tape tab. In this case it may be advantageous to add a film or fibrous material to limit the extension, as described, e.g., in WO 01/68025. Alternatively, it may be advantageous to use a non-elastic material for the inner tab portion, like a soft non-woven material. The proximal and distal end portions are preferably formed of a non-woven material, although in some instances film tapes may be advantageous. Furthermore, the closure tape tab of the present invention may comprise a mechanical fastener component and/or a fingerlift on the distal end portion.

In accordance with a further aspect of the present invention there is provided a prelaminated closure tape, preferably in a stable roll, from which closure tape tabs as described above can be cut. A particularly advantageous feature of the present invention is that due to the closure tape tab construction using an anti-adhesive means the roll can be level-wound, thus considerably increasing the storage capacity on a roll. Without the anti-adhesive means these level wound rolls would block because the adhesive provided on the proximal and/or distal end portions and/or the optional release tape of the fastening tape tab would adhere to the first surface of the inner tab portion of the underlying winding of fastening tape. Planetary winding of the closure tape is of course also feasible.

A further aspect of the present invention relates to a method of manufacturing a closure tape from which closure tape tabs for an absorbent article, particularly for a disposable diaper, can be cut so that the closure tape tabs can be used for fastening of the article on the body of a person. The method comprises the steps of providing a first tape section forming a proximal end portion and a second tape section forming the distal end portion of the closure tape tab; connecting the first and second tape sections by means of an inner tab portion, wherein the inner tab portion has a first major surface and an opposite second major surface and the proximal and distal end portions are connected to the inner tab portion at the first major surface thereof such that opposing ends of the proximal and distal end portions are spaced from each other; and providing an anti-adhesive means at at least part of the first major surface of the inner tab portion in the space.

Another aspect of the invention concerns a method of manufacturing a closure tape from which closure tape tabs for an absorbent article, particularly for a disposable diaper, can be cut so that the closure tape tabs can be used for fastening on the body of a person, which method comprises the steps of providing a continuous tape having at least a first tape section forming a proximal end portion of the closure tape tab and a second tape section forming a distal end portion of the closure tape tab; and connecting to the first and second tape sections an inner tab portion, which inner tab portion has a first major surface and an opposite second major surface. The proximal and distal end portions are connected to the inner tab portion at the first major surface thereof. The continuous tape provides an anti-adhesive means for said inner tab portion on the first major surface thereof, and the continuous tape is provided with at least one line of weakness in the area of the inner tab portion.

In accordance with the latter method it may be preferred to provide two lines of weakness in the continuous tape. A first line of weakness may be provided between the the proximal end portion and an intermediate part, and a second line of weakness may be provided between the distal end portion and the intermediate part. These lines of weakness may be provided in the form of perforations, slits or the like. With this construction the intermediate part can be removed from the inner tab portion. However, one line of weakness is generally sufficient for the closure tape tab to function since upon breaking or opening of the line of weakness the preferred elastic function of the inner tab portion can be achieved. Another advantage of this construction is that no waste material must be removed and disposed.

In accordance with a further aspect of the present invention there is provided a method of manufacturing a closure tape tab for an absorbent article, particularly for a disposable diaper, for fastening of the article on the body of a person. The method comprises the steps of providing a first tape section forming a proximal tape portion and a second tape section forming a distal end portion of the closure tape tab; connecting the first and second tape sections by means of an inner tab portion, wherein the inner tab portion has a first major surface and an opposite second major surface, the proximal end portion is connected to the inner tab portion at the second major surface thereof such that the proximal end portion extends over at least a substantial portion of the inner tab portion, and the distal end portion is connected to the inner tab portion at the first major surface thereof such that at least a portion thereof remains exposed; and providing an anti-adhesive means at at least a part of the exposed portion of the first major surface.

In accordance with all of the above-mentioned methods of manufacturing closure tape tabs, the connection between the inner tab portion and the proximal and distal end portions is preferably made by ultrasonic welding, cold bonding or thermal pressure bonding, or adhesive bonding. Furthermore, it is preferred to provide the anti-adhesive means after the above-mentioned connecting step in the form of a coating, preferably a silicon coating. Alternatively, the anti-adhesive means may be provided in the form of an anti-adhesive paper, anti-adhesive film, a cover strip, a liner or the like.

As already noted above, the present invention is particularly advantageous from a manufacturing point of view since the closure tape tab of the present invention is easy to handle, comprises only a few elements and manufacturing steps and can be stored in a stable roll. A particular advantage of the closure tape tab of the present invention is that it can be level-wound to a roll on which considerably more closure tape can be stored than on conventional planetary wound rolls. The storage capacity is furthermore increased because of the thin construction of the closure tape tab of the present invention.

### Brief Description of the Drawings

In the following preferred embodiments of the present invention will be described in connection with the drawings, in which:
- Fig. 1: is a side view of one embodiment of a fastening tape tab according to the present invention comprising a cover strip;
- Fig. 2: is a side view of another embodiment of a closure tape tab of the present invention similar to Fig. 1, wherein a cover strip is provided only partially within the space between the proximal and distal end portions;
- Fig. 3: is a side view of another embodiment of a closure tape tab of the present invention;
- Figs. 4-6: show an example of a winding sequence for the preparation of a pre-laminated closure tape in roll form, wherein Fig. 4 shows the closure tape tab in a side view without an anti-adhesive means, Fig. 5 shows the closure tape tab of Fig. 4 with an anti-adhesive means which substantially covers the space between the proximal and distal end portions, and Fig. 6 shows how the various layers of the pre-laminated closure tape are level-wound one on top of another;
- Figs. 7-9: show a winding sequence similar to the one illustrated in Figs. 4 to 6, wherein the pre-laminated closure tape is provided with a release tape;
- Fig. 10: is a schematic top view of a level wound roll after winding the closure tape to the roll;
- Fig. 11A: is a schematic side view showing how a closure tape tab of the present invention is applied to a diaper ear;
- Fig. 11B: is a side view similar to Fig. 11A in which the distal end portion of the closure tape tab is folded over to the inside of the diaper;
- Fig. 12A: is a side view similar to Fig. 11A showing how the closure tape tab is applied onto a diaper ear using a release tape;
- Fig. 12B: is a side view similar to Fig. 12A, wherein the distal end portion of the closure tape tab is folded over the inside of the diaper;
- Fig. 13A: is a side view showing how a closure tape tab similar to the one shown in Fig. 3 is applied to a diaper ear;
- Fig. 13B: is a side view similar to Fig. 13A in which the distal end portion is folded over to the inside of the diaper;
- Fig. 14: is a schematic illustration of a coating station for applying an anti-adhesive means in the form of a release coating;
- Fig.15: is a side view of an embodiment of a closure tape tab comprising an anti-adhesive means in the form of a release coating;
- Fig. 16a: is a schematic side view of another embodiment of the closure tape tab of the present invention in which the anti-adhesive means is provided as an integral element with the proximal and distal end portions, wherein perforations are provided to allow removal of the anti-adhesive means from the inner tab portion;
- Fig. 16b: is a schematic side view of an embodiment similar to the one illustrated in Fig. 16a;
- Fig. 17: is a side view of another embodiment of a closure tape tab of the present invention similar to the embodiment of Fig. 3; and
- Fig. 18: is a side view of a further embodiment of a closure tape tab of the present invention similar to the embodiment of Fig. 17.

### Detailed Description of the Preferred Embodiments

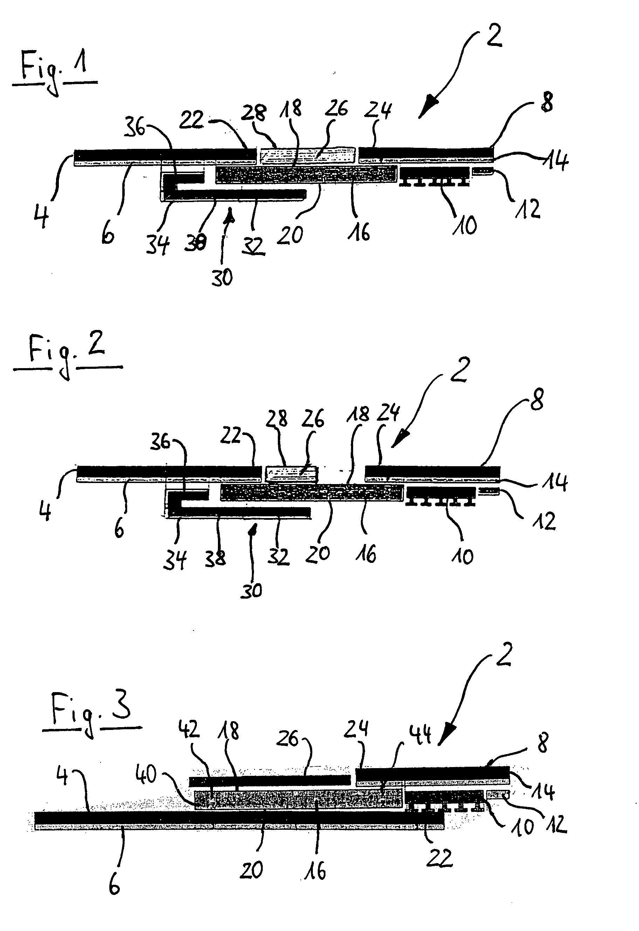

A first embodiment of a closure tape tab 2 is illustrated in Fig. 1. The closure tape tab 2 is particularly useful for a disposable diaper to which it can be connected at a proximal end portion 4. Typically, the closure tape tab 2 is secured to the diaper by means of an adhesive 6 provided on the lower surface of the proximal end portion 4. The adhesive 6 is preferably provided in the form of an adhesive layer extending substantially along the whole length of the proximal end portion 4, wherein the adhesive layer may be continuous or discontinuous. Furthermore, the adhesive layer 6 may be applied in the form of a pattern on the lower surface of the proximal end portion 4.

The closure tape tab 2 furthermore comprises a distal end portion 8 opposite to the proximal end portion 4. The distal end portion 8 constitutes the fastening portion of the closure tape tab 2. To this end, the distal end portion 8 comprises an appropriate fastening means, e.g., a pressure sensitive adhesive layer or, as illustrated in Fig. 1, a mechanical fastener element 10. Preferably, the mechanical fastener element 10 is provided in the form of a strip with upstanding hooks that are adapted to engage with a loop material provided on a diaper landing area. Optionally, the distal end portion 8 comprises finger lift 12 adjacent to the fastening means 10 so as to allow gripping and opening of the closure tape tab 2. The mechanical fastener element 10 and the finger lift 12 are connected to the distal end portion 8 preferably by means of an adhesive layer 14. The adhesive layer 14 is provided on the lower surface of the distal end portion 8 and extends preferably along the whole length thereof. As in the case of the adhesive layer 6 on the proximal end portion 4, the adhesive layer 14 of the distal end portion 8 may be continuous or discontinuous, and may be provided in the form of a pattern.

The proximal end portion 4 and the distal end portion 8 are connected by an inner tab portion 16 having an upper first major surface 18 and a lower second major surface 20. As can be seen in Fig. 1, the proximal and distal end portions 4, 8 are connected to the inner tab portion 16 at the first major surface 18 thereof such that opposing ends 22 and 24 of the proximal and distal end portions 4, 8, respectively, are spaced apart from each other.

While the proximal and distal end portions 4, 8 are typically made of an inelastic material, e.g., a non-woven material, the inner tab portion 16 preferably has some elasticity. For example, the inner tab portion may be provided as a laminate comprising an elastic material and an inelastic material limiting the extensibility of the elastic material.

Furthermore, the closure tape tab 2 of the embodiment shown in Fig. 1 comprises an anti-adhesive means 26 in the form of a cover strip (e.g., liner, anti-adhesive paper, anti-adhesive film). The cover strip 26 shown in Fig. 1 is provided on the first major surface 18 of the inner tab portion 16 and substantially covers the space provided between the opposing ends 22 and 24 of the proximal and distal end portions 4, 8, respectively. Preferably, the top surface 28 of the cover strip 26, i.e., the surface opposite the inner tab portion 16, comprises a low adhesive backsize coating (LAB) which may be provided in the form of a silicon coating. It is also preferred that the upper surfaces of the proximal and distal end portions 4, 8, i.e., the surfaces opposite the adhesive layers 6, 14, respectively, be coated with a LAB, typical in the form of a silicon coating.

Furthermore, it may be advantageous to provide the anti-adhesive means 26 not only in the space between the opposing ends 22 and 24 of the proximal and distal end portions 4, 8 but to extend the anti-adhesive means 26 over these ends 22, 24 as shown and described in more detail in connection with the embodiment illustrated in Fig. 15.

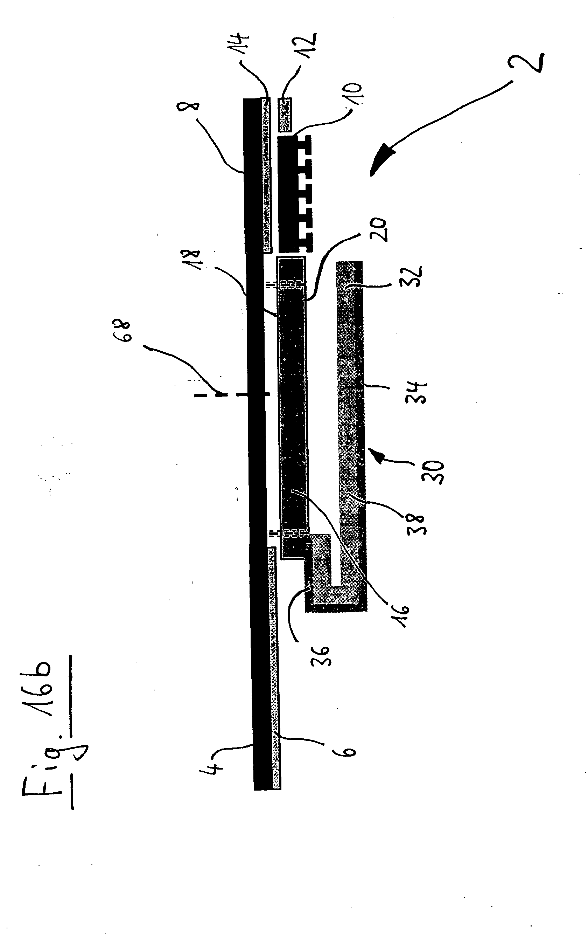

Optionally, the closure tape tab 2 of the present invention comprises a release tape 30 attached to the proximal end portion 4 as shown in Fig. 1. Alternatively, the release tape 30 may be attached to the inner tab portion 16 (see Fig. 16). The release tape 30 typically comprises a backing 32 coated on one surface with an adhesive layer 34. Generally, the release tape 30 is attached to the proximal end portion 4 relatively close to the proximal end of the inner tab portion 16 along a relatively short portion 36 of the release tape 30. The remaining longer portion 38 of the release tape is folded back onto the portion 36 and the inner tab portion 16 so that the adhesive layer 34 is exposed as illustrated in Fig. 1. The release tape 30 assists in securing the closure tape tab 2 to a diaper, wherein the adhesive layer 6 is attached to one surface of the diaper, e.g., the outer surface, and the portion 38 of the release tape 30 is folded over the opposite side of the diaper, e.g., the inner surface thereof, so as to provide a so-called Y-bond.

The closure tape tab 2 of the present invention is typically provided in the form of a closure tape from which the closure tape tabs 2 can be cut transversally. The closure tape itself may be wound in the form of a roll for storage purposes. A particular advantage of the present invention is that the closure tape tab 2 can be provided as a prelaminated closure tape in a stable level-wound roll from which the closure tape tabs 2 can be cut. Level winding is an advantageous process for storing large quantities of tape material in roll form. The anti-adhesive means 26 allows for level winding and the use of a relatively short release tape since there is no more danger of adhesive contacting the first major surface 18 of the inner tab portion 16 upon winding. The anti-adhesive means 26 may be removed from the closure tape of the present invention before the closure tape tabs 2 are cut therefrom. In other words, the anti-adhesive means which is provided in the embodiment of Fig. 1 in the form of a cover strip 26 may be delaminated from the underlying inner tab portion 16 before the closure tape is cut into individual closure tape tabs. Insofar, Fig. 1 can be considered to be a cross-sectional view of the closure tape before it is cut into tape tabs.

Fig. 2 shows another embodiment of a closure tape tab 2 of the present invention. This closure tape tab is essentially identical to the closure tape tab of the embodiment shown in Fig. 1 with the difference that the anti-adhesive means is provided as a cover strip 26 extending only over a part of the first major surface 18 of the inner tab portion 16 in the space between the opposing ends 22, 24 of the proximal and distal end portions 4, 8, respectively. More precisely, the cover strip 26 extends in the space from adjacent the end 22 of the proximal end portion 4 towards the opposing end 24 of the distal end portion 8. However, it does not cover substantially the whole space as the embodiment shown in Fig. 1. Preferably, the cover strip 26 extends in the space along the first major surface 18 at least as far from the end 22 of the proximal end portion 4 as the longer portion 38 of the release tape 30 extends on the second surface 20 of the inner tab portion 16. This closure tape construction is particularly useful for planetary winding of the closure tape tab since the cover strip 26 covers at least that portion of the inner tab portion 16 which could be contacted upon winding by the exposed adhesive 34 of the release tape 30. The upper surface of the proximal end portion 4 is typically also coated with LAB in the form of a silicon coating. An LAB coating can also be applied on the upper surface of the distal end portion 8.

For planetary wound rolls, the cover strip 26 may be applied in the space provided on the first major surface 18 of the inner tab portion 16 during winding from above or below. In other words, the cover strip 26 may be provided either directly in the space or at the opposite side of the tape (e.g., on the release tape30) so that upon winding the cover strip 26 will be arranged in the space of a preceding of following winding of the roll.

Another embodiment of the closure tape tab of the present invention is shown in Fig. 3. The closure tape tab 2 of this embodiment also comprises a proximal end portion 4 and a distal end portion 8 which are both coated with an adhesive layer 6 and 14, respectively. The proximal end portion 4 and the distal end portion 8 are connected by the inner tab portion 16. In accordance with this embodiment, the proximal end portion 4 is connected to the inner tab portion 16 at the second major surface 20 thereof such that the proximal end portion 4 extends over at least a substantial portion of the inner tab portion 16. More preferably, the inner tab portion 16 is attached to the proximal end portion 4 so that the distal end 22 of the proximal end portion 4 extends beyond the inner tab portion 16. The attachment of the inner tab portion 16 to the proximal end portion 4 is provided in accordance with this embodiment at a proximal end 40 thereof. The bonding is schematically indicated at reference sign 42. Preferably, the connection between the inner tab portion 16 and the proximal end portion 4 is made by ultra-sonic welding, but cold or thermo-pressure bonding or adhesive bonding would also be feasible. Connecting the inner tab portion 16 onto the proximal end portion 4 of the closure tape tab 2 only at the proximal end 40 of the inner tab portion 16, while the proximal end portion 4 extends further along the inner tab portion 16, has the advantage that the proximal end portion 4 can also function as a release tape. Thus, in order to attach the closure tape tab 2 to a diaper the proximal end portion 4 is attached to one surface of a diaper end portion so that the edge of the diaper end portion lies approximately at the connection 42 between the proximal end portion 4 and the inner tab portion 16. The remaining (distal) part of the proximal end portion 4 is folded back onto the opposite surface of the diaper end portion so as to create a Y-bond.

The distal end portion 8 of the closure tape tab 2 of the embodiment shown in Fig. 3 is attached to the inner tab portion 16 at the first major surface 18 thereof such that at least a portion of the inner tab portion 16 remains exposed. The attachment between the distal end portion 8 and the inner tab portion 16 can be made, e.g., by ultra-sonic welding, cold or thermo-pressure bonding, adhesive bonding or the like. In Fig. 3 the bonding is illustrated at reference sign 44 as an ultrasonic bonding. A mechanical fastener element 10 is provided on the lower surface of the distal end portion 8 as in the other embodiments.

Furthermore, the closure tape tab 2 of the embodiment shown in Fig. 3 comprises an anti-adhesive means 26 which is provided on at least a part of the exposed portion of the first major surface 18 of the inner tab portion 16. In Fig. 3 the anti-adhesive means 26 covers substantially the entire exposed surface of the inner tab portion 16. The anti-adhesive means 26 may also be provided only along a certain width thereof. For example, if the distal end 22 of the proximal end portion 4 was located underneath the exposed portion of the first major surface 18 of the inner tab portion 16, it would be sufficient to provide the anti-adhesive means 26 only as far as the proximal end portion 4 extends. This would, however, only allow a planetary winding of the closure tape. If level winding is performed, it is preferred to cover the whole exposed portion of the first surface 18 of the inner tab portion 16 with the anti-adhesive means 26.

In accordance with this embodiment it is preferable to temporarily secure the anti-adhesive means 26 to the inner tab portion 16 to maintain its position during further processing. For example, in the case of a cover strip functioning as an anti-adhesive means, the cover strip 26 can be bonded to the inner tab portion 16 by means of ultra-sonic bonding, gluing, application of wax, heat pressing or any other suitable means for bonding. Temporarily securing the anti-adhesive means 26 to the inner tab portion 16 could also be advantageous for the other embodiments of the invention.

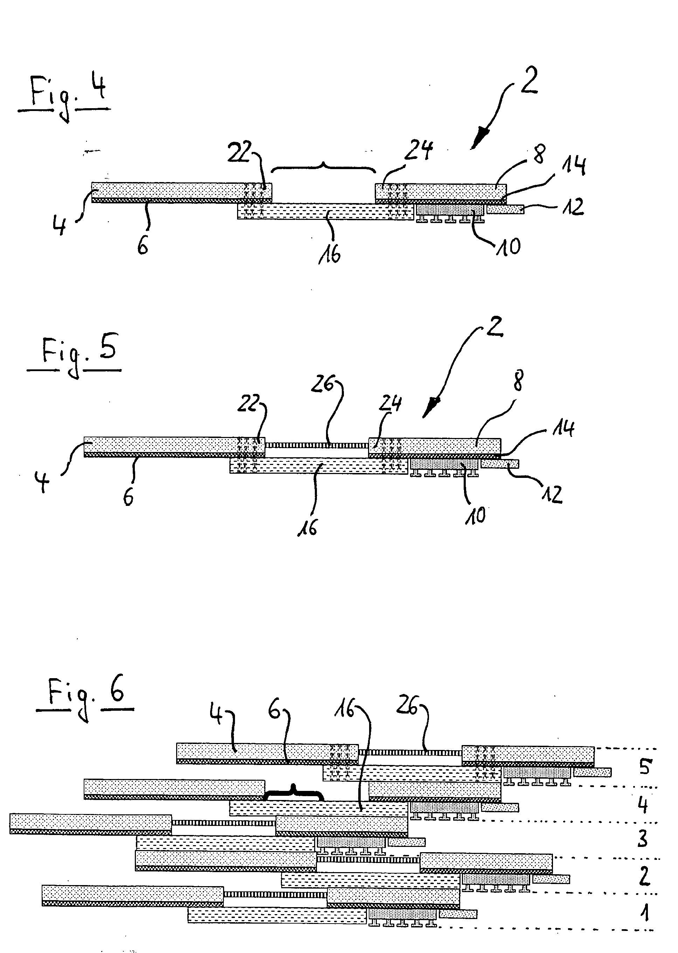

Figs. 4 to 6 illustrate how a closure tape tab being provided as a prelaminated closure tape can be level-wound. The closure tape shown in Figs. 4 to 6 correspond to the embodiment of the closure tape tab 2 shown in Fig. 1, however, without a release tape 30. Furthermore, in Fig. 4 the connection between the proximal end portion 4 and the inner tab portion 16 as well as the connection between the distal end portion 8 and the inner tab portion 16 is made by ultrasonic bonding as schematically illustrated by means of the dashed lines. Thermobonding, adhesive bonding are also possible.

In Fig. 4, the tape tab is illustrated without an anti-adhesive means 26, i.e., in the form the closure tape tab may actually be applied to the diaper. In Fig. 5 the closure tape tab 2 is illustrated with the anti-adhesive means 26 covering substantially the complete space inbetween the opposing ends 22 and 24 of the proximal and distal end portions 4, 8. The anti-adhesive means 26 may be provided in the form of a cover strip, an anti-adhesive paper, an anti-adhesive film or a liner. Alternatively, the anti-adhesive means 26 may be provided in the form of an anti-adhesive coating, preferably a silicon coating. Such an anti-adhesive coating can be used in any of the embodiments of the present invention.

In Fig. 6 it is schematically illustrated how the various layers of prelaminated closure tape according to the present invention can be arranged if level-winding is applied to prepare a roll. As can be seen from Fig. 6, when level-winding the prelaminated closure tape, the various layers or windings, five of which are shown in Fig. 6, are arranged one on top of the other. In winding #4 shown in Fig. 6 it is indicated by means of the bracket in which region the adhesive layer 6 of the uppermost winding #5 would contact the inner tab portion 16 of winding #4 if no anti-adhesive means 26 was present. Accordingly, by means of the anti-adhesive means 26 level winding of the closure tape tab of the present invention can be conducted.

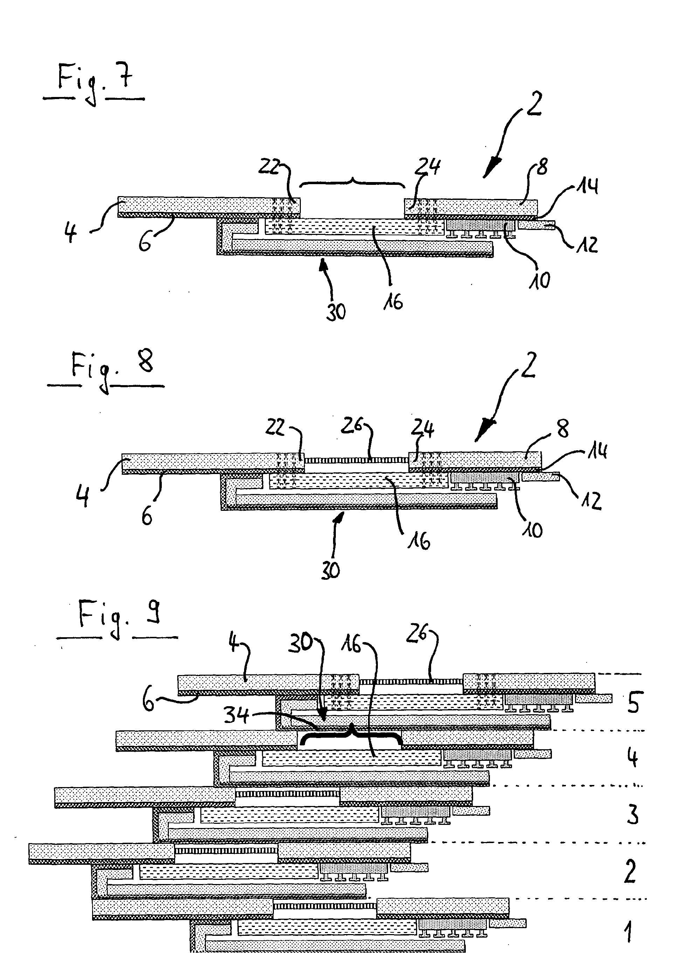

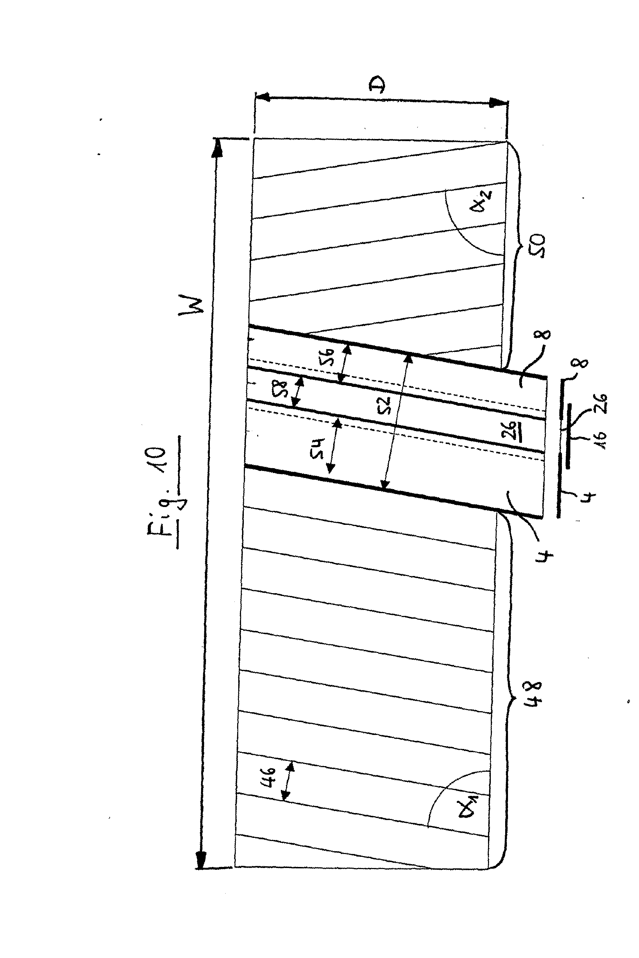

Figs. 7 to 9 show a winding sequence similar to the one illustrated in Figs. 4 to 6, wherein the prelaminated closure tape is provided with a release tape 30. Also in this case it can be seen that upon level-winding of the prelaminated closure tape the adhesive layer 34 of the release tape 30 would contact the inner tab portion 16 if no anti-adhesive means 26 was present. The various layers or windings of an exemplarily illustrated level wound roll of prelaminated closure tape according to the present invention is shown in Fig. 9. By means of the bracket in winding #4 it can be seen how the adhesive layer 34 of the release tape 30 of winding #5 of the closure tape would contact the inner tab portion 16 of the underlying winding #4 of the closure tape of the present invention if no anti-adhesive means 26 was present.

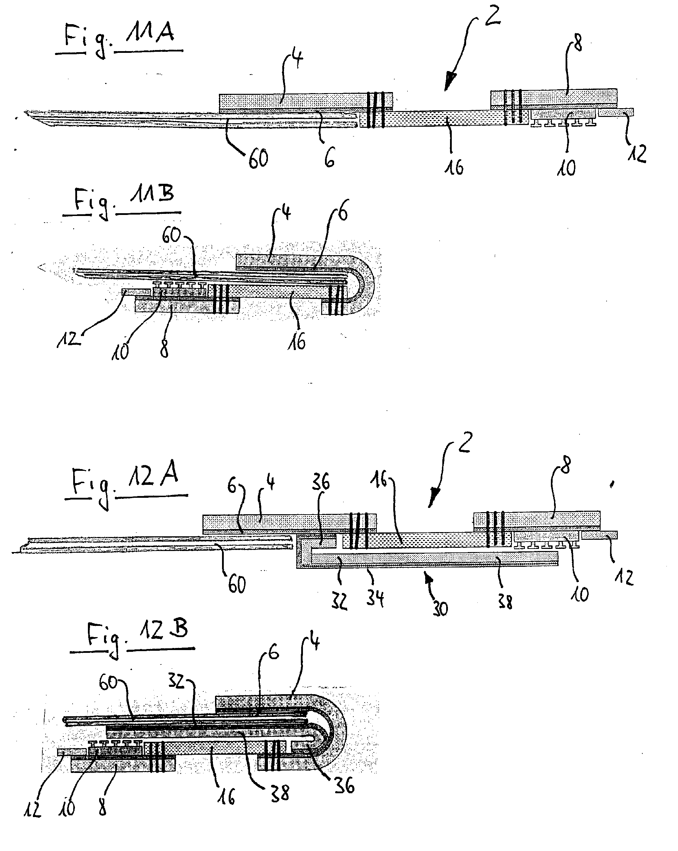

Fig. 10 is a top view of a level-wound roll of a prelaminated closure tape of the present invention. The roll has a diameter D and a width W. The prelaminated closure tape is level-wound by moving the roll sideways or in cross-direction relative to the direction of movement of the closure tape. Oscillating the roll forth and back results in a level-wound roll of prelaminated closure tape, wherein adjacent windings along the width of the roll partially overlap. Thus, only a portion of the closure tape illustrated in Fig. 10 by means of arrow 46 remains visible in the top view. Instead of moving the roll in its axial direction, i.e., in cross-direction to the movement of the closure tape, it would also be possible to have a fixed roll and an oscillating tape feeding mechanism.

As can be seen in Fig. 10, the prelaminated closure tape of the invention is wound on the roll over the whole width in a first direction under an angle α1, a portion of this winding being visible and indicated in Fig. 10 by bracket 48. When this winding reaches the edge of the roll, the direction of roll oscillation changes, or the feeding mechanism changes the direction of movement, so that the tape is wound on the roll under an angle α2 as a successive winding. This winding is illustrated in Fig. 10 by means of bracket 50.

Between brackets 48 and 50 the prelaminated closure tape of the present invention is illustrated. As can be seen, the prelaminated closure tape has a width 52, i.e., the length of the closure tape tab 2 which is generally in the range of between 30 to 200 mm, preferably between 50 to 120 mm and most preferably between 60 to 80 mm. The length 54 of the proximal end portion 4 is typically in the range of between 15 to 80 mm, preferably between 20 to 60 mm, and more preferably between 25 to 40 mm. The length 56 of the distal end portion 8 is typically in the range of between 10 to 70 mm, preferably between 15 to 50 mm, and more preferably between 17 to 30 mm. The length 58 of the space between the opposing ends 22 and 24 of the proximal and distal end portions is typically in the range of 5 to 50 mm, preferably 5 to 40 mm and more preferably 5 to 20 mm.

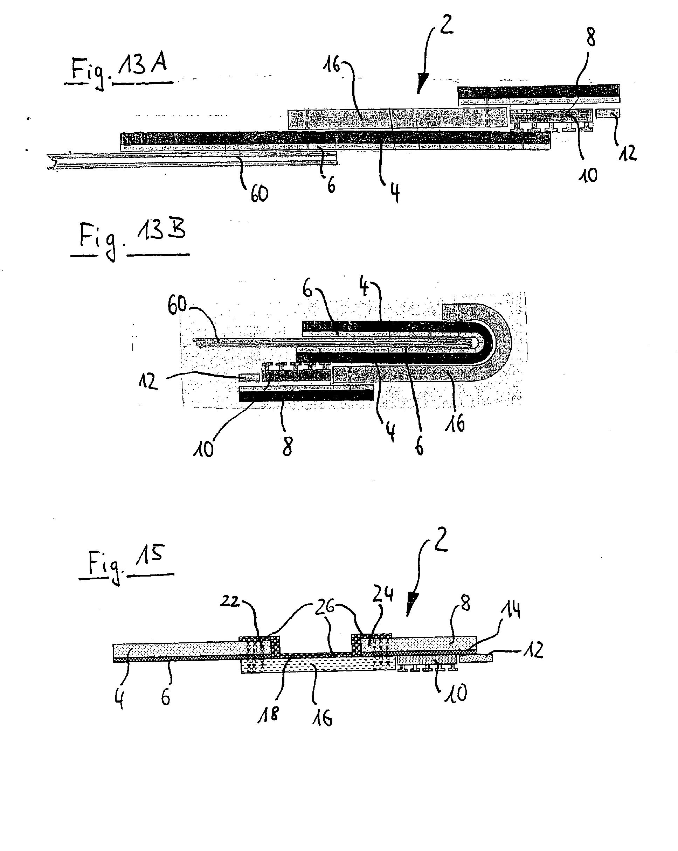

In Fig. 11A it is illustrated how the closure tape tab 2 of the present invention can be attached to an end portion 60 of a diaper. The closure tape tab 2 corresponds to the embodiment shown in Figs. 1 and 2, wherein no release tape 30 is used. Before cutting the closure tape tab 2 from the prelaminated closure tape being unwound from the roll the cover strip 26 functioning as an anti-adhesive means may be removed, e.g., by suction. If the prelaminated closure tape is level-wound on the roll, it is preferred to stabilize the oscillating laminate coming from the level-wound roll during unwinding, e.g., by running the web through two rods and turning the web around 90° thereby. After passing the rods, the web will be turned back to its original configuration. Furthermore, after stabilizing the closure tape, the cover strip 26 can be removed by means of, e.g., a Venturi nozzle, a slipping drive wheel or any other suitable means for removing a liner from a tape laminate. For example, the tape could be guided over a sharp edge or the like. If the anti-adhesive means is provided in the form of an anti-adhesive coating, this can be kept on the first major surface 18 of the inner tab portion 16 so that the additional step of removing a cover strip can be avoided.

As can be seen in Fig. 11A and 11B, the proximal end portion 4 is attached to the end portion 60 of the diaper by means of the adhesive layer 6. Any other suitable means for connecting the proximal end portion 4 to the end portion 60 of the diaper can be used, e.g., ultrasonic bonding, cold or thermo-pressure bonding or adhesive bonding. After attachment of the proximal end portion 4 to the diaper the protruding portion is folded to the inside of the diaper such that the fastening means 10 of the distal end portion 8 of the closure tape tab 2 does no longer protrude from the end portion 60. Preferably, an anti-flagging means is provided for securing the distal end portion 8 in this configuration. For example, it would be possible to use the fastening means 10 to temporarily attach the distal end portion 8 in the configuration shown in Fig. 11 B. When the diaper is to be used, the closure tape tab can be opened by means of the fingerlift 12 and folded in the configuration of Fig. 11A so that the diaper can be fastened to a person.

In Figs. 12A and 12B the attachment of a closure tape tab 2 of the present invention to a diaper is illustrated, wherein a release tape 30 is used. Thus, the closure tape tab 2 shown in Figs. 12A and 12B corresponds to those of Figs. 1 and 2. In accordance with this embodiment, the closure tape tab 2 is attached to the end portion 60 of the diaper by means of the adhesive layer 6 of the proximal end portion 4 and the adhesive layer 34 of the release tape 30. Initially, the cover strip 26 may be removed as described above in connection with Figs. 11A and 11B. Then, the proximal end portion 4 is attached to the outside surface of the diaper by means of the adhesive layer 6, whereupon the longer leg 38 of the release tape 30 is folded over the inside surface of the end portion 60 of the diaper and adhered thereto by means of the adhesive layer 34. Then, the protruding portion of the closure tape tab 2 is folded to the inside of the diaper and preferably secured in the configuration shown in Fig. 12B by means of an anti-flagging feature. Alternatively, the distal end of the tape tab is already previously connected to the release tape by an anti-flagging means so that the protruding portion of the tape tab 2 can be folded to the inside of the diaper simultaneously with the release tape 30.

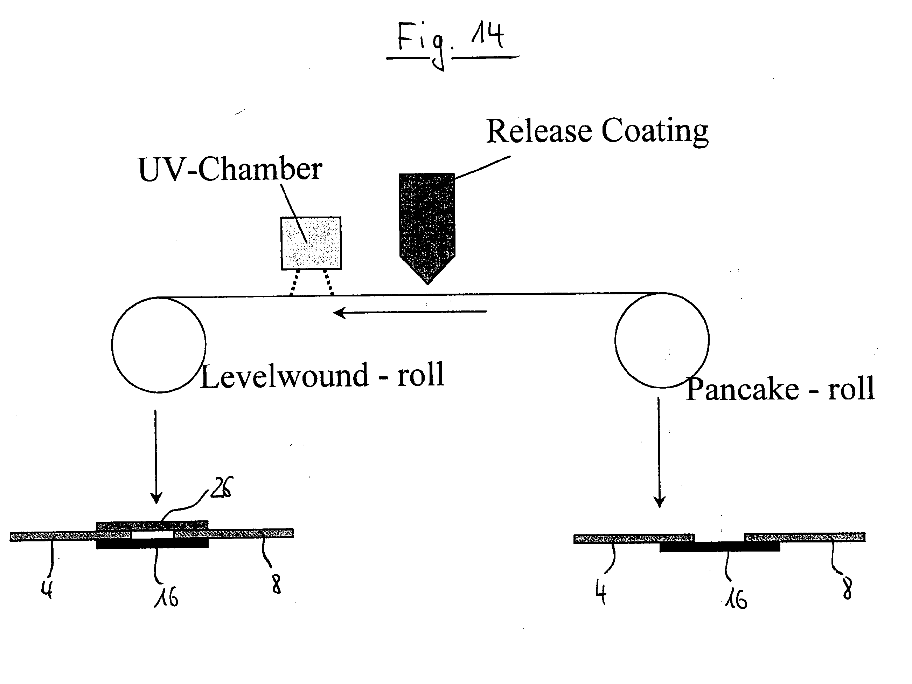

In Figs. 13A and 13B it is illustrated how the closure tape tab 2 of the embodiment shown in Fig. 3 can be applied to a diaper end portion 60. After removing the cover strip 26, the closure tape tab 2 is attached to the diaper end portion 60 by means of the adhesive layer 6 of the proximal end portion 4. However, in accordance with this embodiment only a part of the proximal end portion 4 is attached to the outer face of the diaper end portion 60 and the remaining and protruding proximal end portion 4 is folded around the end portion 60 to create a Y-bond as shown in Fig. 13B. The protruding part of the closure tape tab laminate 2, i.e., the inner tab portion 16 and distal end portion 8, are folded to the inside of the diaper and preferably secured in this configuration by means of an anti-flagging feature.

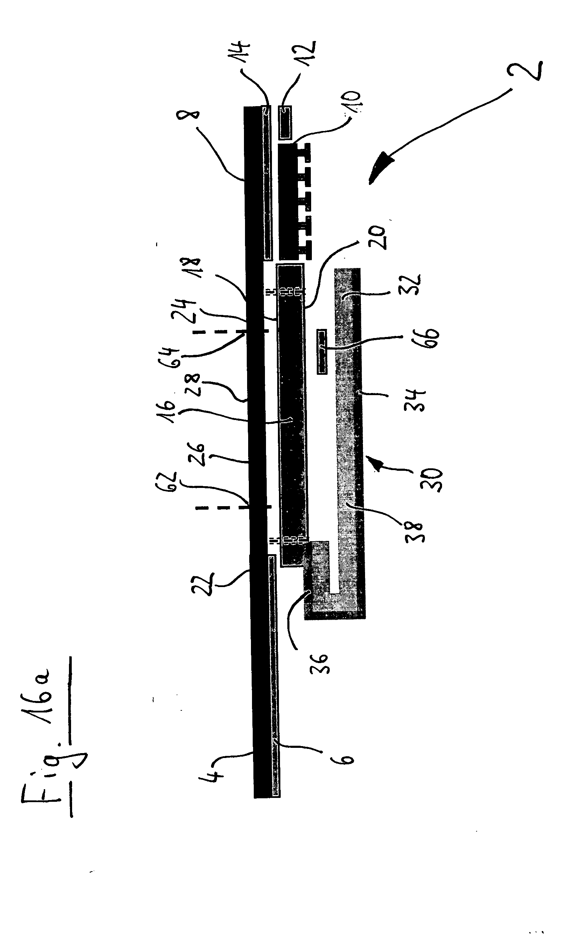

In Fig. 14, a schematic illustration for a coating process for release coating the first major surface 18 of the inner tab portion 16 is illustrated. As can be seen in Fig. 14, a closure tape comprising sections which later form a proximal end portion 4, a distal end portion 8 and an inner tab portion 16 of the closure tape tab connecting the proximal and distal end portions 4, 8 is wound on a "pancake roll", i.e., a planetary wound roll. From this pancake roll the closure tape is unwound and fed to a release coating station where a release coating in form of a low adhesive back size coating functioning as an anti-adhesive means is provided for at least a part of the first major surface 18 of the inner tab portion 16 in the space between the opposing ends 22 and 24 of the proximal and distal end portions 4, 8, respectively. Typically, the space is completely coated with the release coating, and even more preferably the release coating is applied over the ends 22 and 24 of the proximal and distal end portions 4, 8, respectively, as schematically shown in Fig. 14. Optionally, a curing station (e.g., a UV-chamber) is provided for curing the release coating on the closure tape. After curing the prelaminated closure tape is wound onto a roll, preferably by means of a level winding process. In the schematic illustration of Fig. 14, the closure tape is also shown schematically only. As described above and below, the closure tape provided on the pancake roll may comprise further elements such as a fastening means, a fingerlift, etc.

In Fig. 15 a more detailed illustration of the closure tape tab 2 according to this embodiment of the present invention is illustrated. Also in this embodiment, the closure tape tab 2 comprises a proximal end portion 4 and a distal end portion 8 which are connected by means of the inner tab portion 16. As in the other embodiments, the inner tab portion 16 forms a bridge, preferably an elastic bridge in order to provide a more comfortable closure tape tab. Furthermore, also in accordance with this embodiment a release tape may be used for attachment of the closure tape tab 2 to the diaper. The proximal end portion 4 and the inner tab portion 16 as well as the distal end portion 8 and the inner tab portion 16 are connected by ultra-sonic bonding or equivalent thermobonding etc., as schematically shown by the dashed lines. In accordance with this embodiment of the closure tape tab 2 the release coating 26 forming the anti-adhesive means is applied over the exposed first major surface 18 of the inner tab portion 16 and the opposing ends 22 and 24 of the proximal and distal end portions, respectively. This construction has the additional advantage that a release coating is applied over potentially ultra-sonically damaged or destroyed zones of the proximal and distal end portions 4, 8, respectively. This may be particularly advantageous if non-woven materials are used for the proximal and distal end portions. Typically, the ultra-sonic bonding destroys part of the LAB coating applied to the proximal and distal end portions 4, 8 so that the release properties of those portions are affected. Overcoating these potentially damaged bonding areas with a release coating secures appropriate release properties. The coating process can be done, e.g., by gravure-type coating methods, heavy duty multi-roll coaters or by spray coating devices.

Fig. 16a shows another embodiment of the closure tape tab 2 of the present invention. This embodiment is similar to the embodiment shown in Fig. 1 and comprises a proximal end portion 4, a distal end portion 8, an inner tab portion 16 and an optional release tape 30. The closure tape tab 2 furthermore comprises an anti-adhesive means. In accordance with this embodiment the anti-adhesive means is provided as an intermediate part 26 connecting the proximal end portion 4 and the distal end portion 8. The intermediate part 26 is formed as a continuous tape member (i.e. integrally) with the proximal and distal end portions 4, 8 and is removable from the closure tape tab 2 so as to expose the first major surface 18 of the inner tab portion 16. In other words, the proximal end portion 4, the intermediate part 26 and the distal end portion 8 are made form a single strip of material to which the inner tab portion 16 is attached so as to bridge the intermediate part 26. A line of weakness 62 is provided between the end 22 of the proximal end portion 4 and the intermediate part 26, and another line of weakness 64 is provided between the intermediate part 26 and the end 24 of the distal end portion 8 to facilitate removal of the intermediate part 26 from the closure tape tab 2. For example, the lines of weakness 62, 64 may be provided as perforations, slits or the like.

This embodiment is particularly advantageous from a manufacturing point of view since it does not require the delivery of separate elements for the proximal and distal end portions and the anti-adhesive means but rather uses an integral single strip of material that is provided with lines of weakness. Furthermore, LAB coating may be performed more easily with an integral strip of material than with a plurality of elements.

In the closure tape tab 2 shown in Fig. 16a the release tab 30 is attached to the proximal end of the inner tab portion 16. However, the functioning is similar to that described with respect to the other embodiments.

Furthermore, in the embodiment shown in Fig. 16a an anti-flagging means 66 is provided to secure the release tape 30 to the fastening tape tab 2, particularly to the second major surface 20 of the inner tab portion 16. The anti-flagging means 66 may be provided, e.g., in the form of a hotmelt adhesive or wax which is continuously or spot coated between the inner tab portion 16 and the release tape 30. The hotmelt adhesive or wax can be applied, e.g., by strip, spray, spot, line coating etc. Although shown in Fig. 16a only, this anti-flagging means 66 can also be provided in any of the embodiments of the invention comprising a release tape.

An embodiment similar to the one of Fig. 16a is shown in Fig.16b. In accordance with this embodiment, the closure tape tab 2 comprises a continuous tape member forming the proximal end portion 4 and the distal end portion 8. In the area of the inner tab portion 16, the continuous tape member functions as an anti-adhesive means so that the tape can be wound into a roll. The continuous tape member is provided with a predetermined line of weakness or breaking line 68 which is, for example, a perforated line which may be straight or sinusoidal.

When the tape is unwound and cut to individual closure tape tabs, which can be attached, e.g., to a diaper, the predetermined line of weakness 68 can be broken or opened by the user. In this condition, the closure tape tab 2 has the proximal and distal end portions 4, 8 only connected by the inner tab portion 16. One advantage of this construction is that no waste material is generated which is convenient to the manufacturer.

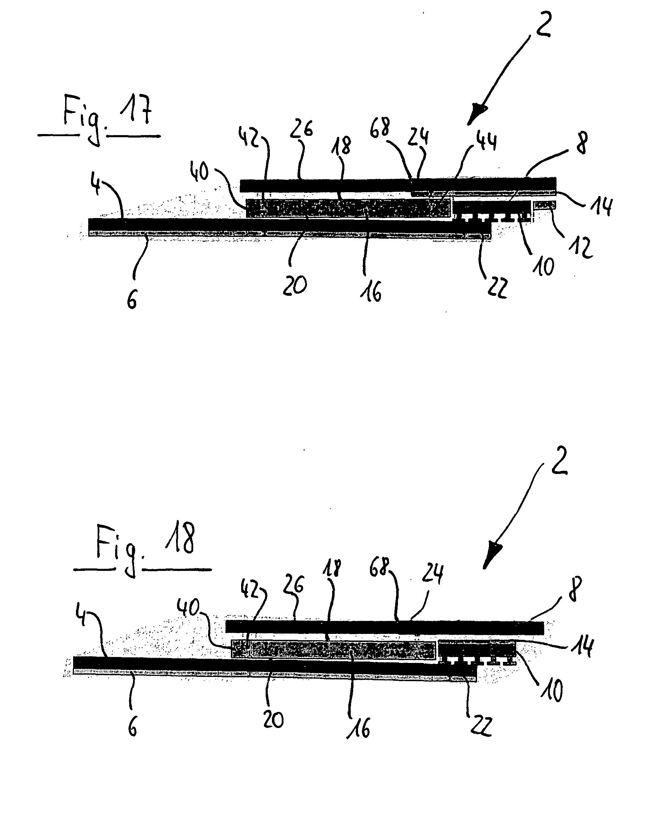

Another embodiment of closure tape tab 2 of the present invention is shown in Fig. 17. This embodiment is generally based on the embodiment shown in Fig. 3. However, in this embodiment the distal end portion 8 and the anti-adhesive means 26 are formed as an integral element as described above in connection with Fig. 16. The anti-adhesive means 26 can be separated from the distal end portion 8 so as to expose the first major surface 18 of the inner tab portion 16. To this end, a line of weakness 68 (e.g., a perforation) may be provided between the distal end of the anti-adhesive means 26 and the proximal end 24 of the distal end portion 8.

As can be seen in Fig. 17, the adhesive layer 14 is provided only beneath the distal end portion 8 of the closure tape tab 2. This may be achieved, e.g., by strip coating the adhesive onto the lower side of the integral element 8, 26.

Another embodiment of a closure tape tab 2 of the present invention is shown in Fig. 18. This embodiment is generally similar to the embodiment of Fig. 17 in that it comprises an integral element for the distal end portion 8 and the anti-adhesive means 26. However, in this embodiment no adhesive is provided on the lower side of the distal end portion 8. Rather, in accordance with this embodiment the adhesive layer 14 is provided on the mechanical fastener element 10. This has the advantage that no strip coating process and no finger lift 12 is required since the lower surface of the distal end portion 8 is free of adhesive.

## Claims

1. A closure tape tab for an absorbent article, particularly for a disposable diaper, for fastening of the article on the body of a person, comprising a proximal end portion and a distal end portion being connected by an inner tab portion, said inner tab portion having a first major surface and an opposite second major surface, wherein said proximal and distal end portions are connected to said inner tab portion at said first major surface thereof such that opposing ends of said proximal and distal end portions are spaced apart from each other, and wherein an anti-adhesive means is provided at at least a part of said first major surface of said inner tab portion in said space.

2. The closure tape tab according to claim 1, wherein said anti-adhesive means extends in said space at least from adjacent the end of said proximal end portion towards the opposing end of the distal end portion.

3. The closure tape tab according to claim 1 or 2, wherein said anti-adhesive means extends at least partially over at least one of said proximal and distal end portions.

4. The closure tape tab according to claim 3, wherein said anti-adhesive means extends at least as far over said proximal and/or distal end portion as said inner tab portion.

5. The closure tape tab according to any of claims 1 to 4, further comprising a release tape attached to said proximal end portion or the second major surface of said inner tab portion, wherein the release tape extends at least partially along the second major surface of said inner tab portion.

6. The closure tape tab according to claim 5, wherein said anti-adhesive means extends in said space along said first major surface at least as far as the release tape extends on said second major surface.

7. The closure tape tab according to any of claims 1 to 6, wherein said anti-adhesive means substantially covers said space.

8. A closure tape tab for an absorbent article, particularly for a disposable diaper, for fastening of the article on the body of a person, comprising a continuous tape member having at least a proximal end portion and a distal end portion, said proximal and distal end portions being connected to an inner tab portion, said inner tab portion having a first major surface and an opposite second major surface, wherein said proximal and distal end portions are connected to said inner tab portion at said first major surface thereof, wherein said continuous tape member provides an anti-adhesive means for said inner tab portion and comprises at least one line of weakness in the area of said inner tab portion.

9. The closure tape tab according to claim 8 comprising two lines of weakness, wherein a first line of weakness is provided between said proximal end portion and an intermediate part and a second line of weakness is provided between said distal end portion and said intermediate part.

10. A closure tape tab for an absorbent article, particularly for a disposable diaper, for fastening of the article on the body of a person, comprising a proximal end portion and a distal end portion being connected by an inner tab portion, said inner tab portion having a first major surface and an opposite second major surface, wherein said proximal end portion is connected to said inner tab portion at said second major surface thereof such that said proximal end portion extends over at least a substantial portion of said inner tab portion, and said distal end portion is connected to said inner tab portion at said first major surface thereof such that at least a portion thereof remains exposed, and wherein an anti-adhesive means is provided at at least a part of said exposed portion of the first major surface of the inner tab portion.

11. The closure tape tab according to claim 10, wherein said anti-adhesive means extends over substantially the entire length of said exposed portion of said inner tab portion.

12. The closure tape tab according to any of claims 1 to 11, wherein said anti-adhesive means is an anti-adhesive paper, an anti-adhesive film, a cover strip or a liner.

13. The closure tape tab according to any of claims 1 to 7, 10 and 11, wherein said anti-adhesive means is an anti-adhesive coating, preferably a silicon coating.

14. The closure tape tab according to any of claims 1 to 13, wherein said inner tab portion comprises an elastic, an elastic/nonwoven composite, or soft nonwoven composites.

15. The closure tape tab according to any of claims 1 to 14, wherein said proximal and distal end portions and/or said proximal and distal end portions and said intermediate part are comprised of a nonwoven material.

16. The closure tape tab according to any of claims 1 to 15, wherein an adhesive and/or a mechanical fastener component and/or a fingerlift are provided on the distal end portion.

17. The closure tape tab according to any of claims 10 to 16, wherein the anti-adhesive means is provided integrally with said distal end portion and is removable from the closure tape tab so as to expose the first major surface of said inner tab portion.

18. The closure tape tab according to claim 17, wherein a line of weakness is provided between the distal end of the anti-adhesive means and the proximal end of said distal end portion.

19. Prelaminated closure tape, preferably in a stable roll, from which closure tape tabs according to any of claims 1 to 18 can be cut.

20. The closure tape according to claim 19 in a roll, wherein said roll is levelwound.

21. A method of manufacturing a closure tape from which closure tape tabs for an absorbent article, particularly for a disposable diaper, can be cut, said closure tape tabs being for fastening of the article on the body of a person, the method comprising the steps of:
providing a first tape section forming a proximal end portion and a second tape section forming a distal end portion of said closure tape tab;
connecting said first and second tape sections by means of an inner tab portion, said inner tab portion having a first major surface and an opposite second major surface, wherein said proximal and distal end portions are connected to said inner tab portion at said first major surface thereof such that opposing ends of said proximal and distal end portions are spaced from each other; and
providing an anti-adhesive means at at least a part of said first major surface of said inner tab portion in said space.

22. A method of manufacturing a closure tape from which closure tape tabs for an absorbent article, particularly for a disposable diaper, can be cut, said closure tape tabs being for fastening of the article on the body of a person, the method comprising the steps of:
providing a continuous tape having at least a first tape section forming a proximal end portion and a second tape section forming a distal end portion of said closure tape tab;
connecting to said first and second tape sections an inner tab portion, said inner tab portion having a first major surface and an opposite second major surface, wherein said proximal and distal end portions are connected to said inner tab portion at said first major surface thereof, wherein said continuous tape provides an anti-adhesive means for said inner tab portion and is provided with at least one line of weakness in the area of the inner tab portion.

23. The method of claim 22 comprising two lines of weakness, wherein a first line of weakness is provided between said proximal end portion and an intermediate part and a second line of weakness is provided between said distal end portion and said intermediate part.

24. A method of manufacturing a closure tape from which closure tape tabs for an absorbent article, particularly for a disposable diaper, can be cut, said closure tape tabs being for fastening of the article on the body of a person, the method comprising the steps of:
providing a first tape section forming a proximal end portion and a second tape section forming a distal end portion of said closure tape tab;
connecting said first and second tape sections by means of an inner tab portion, said inner tab portion having a first major surface and an opposite second major surface, wherein said proximal end portion is connected to said inner tab portion at said second major surface thereof such that said proximal end portion extends over at least a substantial portion of said inner tab portion, and said distal end portion is connected to said inner tab portion at said first major surface thereof such that at least a portion thereof remains exposed; and
providing an anti-adhesive means at at least a part of said exposed portion of the first major surface.

25. The method of any of claims 21 to 24, wherein the connection between said inner tab portion and said proximal and distal end portions is made by ultrasonic welding, cold or thermo pressure bonding, or adhesive bonding, or by any combination of welding/bonding methods.

26. The method of claim 25, wherein after said welding step the anti-adhesive means is provided in the form of a coating, preferably a silicon coating.

27. The method of any of claims 21 to 25, wherein the anti-adhesive means is provided in the form of an anti-adhesive paper, anti-adhesive film, a cover strip or liner.

28. The method of any of claims 24 to 27, wherein the distal end portion and the anti-adhesive means are provided as an integral element, wherein the anti-adhesive means is removable from the distal end portion.
